# EUROPEAN PATENT APPLICATION

(11) **EP 4 675 020 A1**
(43) Date of publication of application: **07.01.2026**
(21) Application number: 24784652.0
(22) Date of filing: 05.03.2024
(51) Int. Cl.: C40B 40/06, C12N 15/09, C12P 21/00, C12Q 1/6809

(54) **METHOD FOR PRODUCING NUCLEIC ACID DISPLAY LIBRARY, SCREENING METHOD, AND METHOD FOR PRODUCING POLYPEPTIDES**

(30) Priority: 03.04.2023 JP 2023060413
(71) Applicant: FUJIFILM Corporation, Tokyo 106-8620 (JP)
(72) Inventor: IZUTA, Shin, Ashigarakami-gun, Kanagawa 258-8577 (JP); ISHII, Yukiko, Ashigarakami-gun, Kanagawa 258-8577 (JP)
(74) Representative: Hoffmann Eitle
(86) International application number: PCT/JP2024/008332
(87) International publication number: WO 2024/209857

(57) **Abstract**

A production method of a nucleic acid display library, including producing a nucleic acid-polypeptide conjugate by expressing a polypeptide from a nucleic acid and linking the nucleic acid to the polypeptide, in which a modification molecule may or may not be present in the nucleic acid-polypeptide conjugate and separating the nucleic acid-polypeptide conjugate from a mixture of a polypeptide not linked to the nucleic acid and the nucleic acid-polypeptide conjugate, independently of a nucleotide sequence of the nucleic acid and the modification molecule; and a screening method and a production method of a polypeptide, in which the nucleic acid display library is used.

## Description

### BACKGROUND OF THE INVENTION

### 1. Field of the Invention

The present disclosure relates to a production method of a nucleic acid display library, a screening method, and a production method of a polypeptide.

### 2. Description of the Related Art

Medium-molecular-weight compounds, including polypeptides, have attracted attention as compounds that possess low manufacturing cost and high stability, which are features of low-molecular-weight compounds, as well as a binding property to target proteins, which is a feature of high-molecular-weight compounds such as antibodies. They have begun to be used in a wide range of bio-related industries, including therapeutic and diagnostic drugs. Among these, special polypeptides that can achieve high binding properties to target proteins and stability by incorporating an unnatural amino acid that does not naturally exist have been actively developed.

As a method for efficiently acquiring a polypeptide having a target activity such as binding property to a target protein, an in vitro molecular evolution method such as an mRNA display method, a cDNA display method, or a ribosome display method is known. The in vitro molecular evolution method is a method in which various polypeptides are linked to nucleic acids encoding the amino acid sequences of the respective polypeptide to form nucleic acid-polypeptide conjugates, and after selecting the nucleic acid-polypeptide conjugates having a target activity against a target substance, the nucleotide sequences of the nucleic acid portions are decoded to identify polypeptides having the target activity.

In a case of producing a nucleic acid-polypeptide conjugate by the above-described method, since the binding efficiency between the polypeptide and the nucleic acid is low, a mixture is obtained in which not only the nucleic acid-polypeptide conjugate but also unlinked components such as an unlinked polypeptide and an unlinked nucleic acid are intermixed.

In WO2005/012902A, biotin and an enzyme cleavage site are introduced into a conjugate of mRNA and puromycin as a linker, the conjugate is immobilized on avidin beads, reverse transcription of nucleic acid is performed, the enzyme cleavage site is cleaved, and Histag purification is performed to obtain an mRNA-polypeptide conjugate.

According to the method of WO2005/012902A, a nucleic acid-polypeptide conjugate from which unlinked components have been removed is obtained.

### SUMMARY OF THE INVENTION

According to the method described in WO2005/012902A, the nucleic acid-polypeptide conjugate can be separated from unlinked components, but the separation requires a multi-step process and is therefore cumbersome. In view of such circumstances, the present disclosure relates to a simple production method of a nucleic acid display library, and a screening method and a method for producing a polypeptide, in which the produced nucleic acid display library is used.

The means for achieving the above-mentioned object include the following aspects.
<1> A production method of a nucleic acid display library, comprising:
   producing a nucleic acid-polypeptide conjugate by expressing a polypeptide from a nucleic acid and linking the nucleic acid to the polypeptide, in which a modification molecule may or may not be present in the nucleic acid-polypeptide conjugate; and
   separating the nucleic acid-polypeptide conjugate from a mixture of a polypeptide not linked to the nucleic acid and the nucleic acid-polypeptide conjugate, independently of a nucleotide sequence of the nucleic acid and the modification molecule.
<2> The production method of a nucleic acid display library according to <1>, in which the separation of the nucleic acid-polypeptide conjugate includes bringing the mixture into contact with a solid-phase carrier capable of binding to the nucleic acid of the nucleic acid-polypeptide conjugate, or adding a solution capable of rendering nucleic acids insoluble.
<3> The production method of a nucleic acid display library according to <1> or <2>, in which the separation of the nucleic acid-polypeptide conjugate includes bringing the mixture into contact with a solid-phase carrier capable of binding to the nucleic acid of the nucleic acid-polypeptide conjugate.
<4> The production method of a nucleic acid display library according to <3>, further comprising:
   eluting the nucleic acid-polypeptide conjugate bound to the solid-phase carrier with water or an aqueous solution.
<5> The production method of a nucleic acid display library according to <4>, in which a total concentration of dissolved substances in the aqueous solution is 0.2 M or less.
<6> The production method of a nucleic acid display library according to any one of <2> to <5>, in which a surface material of the solid-phase carrier is silica, glass, polysaccharides, a carboxy group-containing polymer, a hydroxy group-containing polymer, or hydroxyapatite.
<7> The production method of a nucleic acid display library according to any one of <2> to <6>, in which the solid-phase carrier is magnetic beads.
<8> The production method of a nucleic acid display library according to any one of <2> to <7>, in which the solid-phase carrier is magnetic beads coated with silica or magnetic beads coated with a carboxy group.
<9> The production method of a nucleic acid display library according to any one of <1> to <8>, in which the nucleic acid of the nucleic acid-polypeptide conjugate includes mRNA.
<10> The production method of a nucleic acid display library according to <9>, further comprising:
   producing a conjugate of an mRNA/cDNA double-stranded complex and a polypeptide by reverse transcription of the mRNA.
<11> A screening method comprising:
   producing a nucleic acid display library by the production method of a nucleic acid display library according to any one of <1> to <10>; and
   selecting a nucleic acid-polypeptide conjugate having a target activity from the nucleic acid display library and identifying a nucleotide sequence of a nucleic acid of the selected nucleic acid-polypeptide conjugate.
<12> A production method of a polypeptide, comprising:
   acquiring a polypeptide having the target activity based on a nucleotide sequence identified by the screening method according to <11>.

According to the present disclosure, there are provided a simple production method of a nucleic acid display library; and a screening method and a method for producing a polypeptide, in which the produced nucleic acid display library is used.

### DESCRIPTION OF THE PREFERRED EMBODIMENTS

Hereinafter, embodiments according to the present disclosure will be described. These descriptions and Examples are only illustrative of the embodiments and do not limit the scope of the embodiments. The action mechanism mentioned in the present disclosure includes estimation, and the accuracy thereof does not limit the scope of the embodiments.

The term "step" or a term representing a step in the present disclosure includes not only a step independent of another step but also a step that, even in a case of not being clearly distinguished from another step, achieves the purpose of the step.

In the present disclosure, a numerical range expressed using "to" indicates a range including numerical values before and after "to" as a minimum value and a maximum value.

In the numerical ranges described stepwise in the present disclosure, the upper limit value or the lower limit value described in one numerical range may be replaced with the upper limit value or the lower limit value of the numerical range described stepwise in other stages. In addition, in the numerical ranges described in the present disclosure, the upper limit value or the lower limit value of the numerical range may be replaced with the value shown in Examples.

In the present disclosure, each component may contain a plurality of types of substances corresponding thereto. In the present disclosure, upon referring to an amount of each component in a composition, the amount means a total amount of a plurality of types of substances present in the composition unless otherwise specified, in a case where a plurality of types of substances corresponding to each component are present in the composition.

In the present disclosure, in the notation of an amino acid, the three-letter notation and the one-letter notation established by IUPAC-IUBMB joint commission on Biochemical Nomenclature (IUPAC-IUBMB JCBN) are used. Unless otherwise specified, the amino acid referred to in the present disclosure is an L-amino acid.

In the present disclosure, the unit "M" is a unit of molar concentration and is synonymous with mol/L.

In the present disclosure, unless otherwise specified, even in a case where an element is expressed in a singular form, the presence of a plurality of elements is not excluded unless a technical contradiction occurs.

### <Production method of nucleic acid display library>

The production method of a nucleic acid display library of the present disclosure includes
producing a nucleic acid-polypeptide conjugate by expressing a polypeptide from a nucleic acid and linking the nucleic acid to the polypeptide, in which a modification molecule may or may not be present in the nucleic acid-polypeptide conjugate (hereinafter, this step is also referred to as a "conjugate production step"), and
separating the nucleic acid-polypeptide conjugate from a mixture of a polypeptide not linked to the nucleic acid and the nucleic acid-polypeptide conjugate, independently of a nucleotide sequence of the nucleic acid and the modification molecule (hereinafter, this step is also referred to as a "separation step").

In general, a nucleic acid-polypeptide conjugate in a nucleic acid display library is produced by expressing corresponding polypeptides from a plurality of types of nucleic acids by a polypeptide synthesis system and linking the expressed polypeptides to the nucleic acids. The product obtained by using the polypeptide synthesis system is a mixture in which not only the nucleic acid-polypeptide conjugate but also unlinked components such as an unlinked polypeptide and an unlinked nucleic acid are intermixed. In a case of the method described in WO2005/012902A, the nucleic acid-polypeptide conjugate can be separated from the unlinked components and used in the next step. On the other hand, the inventors have conceived that, according to the production method of a nucleic acid display library of the present disclosure, a nucleic acid display library can be easily produced while maintaining good performance of the nucleic acid display library.

In the unlinked components, particularly, an unlinked polypeptide is present in an amount of several times to several hundred times with respect to the nucleic acid-polypeptide conjugate. In a case of screening a polypeptide having an activity against a target substance, in a case where a large number of unlinked polypeptides are present in the system, the unlinked polypeptides occupy an action site on the target substance, and the nucleic acid-polypeptide conjugate cannot act on the target substance, which may make it impossible to identify a target polypeptide. On the other hand, even in a case where unlinked nucleic acids are intermixed, the nucleic acids do not act on the target substance. Therefore, the performance of the obtained nucleic acid display library is not significantly impaired. Unlike the related art, it is considered that, even in a case where the unlinked polypeptide can be removed by a method of removing the unlinked polypeptide independently of the nucleotide sequence of the nucleic acid and the modification molecule, good performance of the nucleic acid display library can be maintained. According to the production method of a nucleic acid display library of the present disclosure, unlinked polypeptides can be removed easily (for example, only three steps of a solid-phase binding step, a washing step, and an elution step) and in a short time (for example, about 30 minutes) without requiring multiple (for example, eight steps including a binding step to avidin beads, a washing step, a reverse transcription step, an enzymatic cleavage step, a Ni-NTA beads binding step, a washing step, an elution step, and a size exclusion purification step) and long time (3 hours or more) steps or various materials (avidin beads, a reverse transcription enzyme, a cleavage enzyme, Ni-NTA beads, a gel for size exclusion purification, and the like) as in the related art. In addition, since a simple method can be adopted, there is an advantage in terms of cost. In addition, the degree of freedom in the design of the nucleic acid-polypeptide conjugate, such as the design of the modification molecule, is also increased. Furthermore, since the number of steps is small, the loss of the nucleic acid-polypeptide conjugate throughout the entire separation step is small.

In the present disclosure, a polypeptide that is not linked to a nucleic acid is also referred to as an "unlinked polypeptide", and a nucleic acid that is not linked to a polypeptide (including a linker conjugate of a nucleic acid and a linker) is also referred to as an "unlinked nucleic acid".

Hereinafter, the nucleic acid display library to be produced will be described.

### [Nucleic acid display library]

The nucleic acid display library refers to an assembly of a plurality of types of nucleic acid-polypeptide conjugates, each of which is taken as a constituent unit of nucleic acid-polypeptide conjugates. The number of clones and the number of copies of the nucleic acid-polypeptide conjugate constituting the nucleic acid display library are not limited.

The polypeptide in each nucleic acid-polypeptide conjugate is a polypeptide expressed by the nucleic acid to be linked. That is, the nucleic acid and the polypeptide in each nucleic acid-polypeptide conjugate are associated with each other, and the polypeptide can be identified by analyzing the nucleic acid sequence.

The nucleic acid display library is, for example, an mRNA display library, a cDNA display library, or a ribosome display library.

The nucleic acid-polypeptide conjugate is a structure in which a nucleic acid and a polypeptide are linked to each other. The nucleic acid and the polypeptide are preferably linked through a constituent such as a linker or a ribosome.

The nucleic acid of the nucleic acid-polypeptide conjugate is not particularly limited. In the present disclosure, the nucleic acid refers to a molecule that carries information for synthesizing a polypeptide. The nucleic acid is a term including all nucleic acids (for example, DNA, RNA, an analog thereof, a natural product, and an artificial product) and all nucleic acids to which a low-molecular-weight compound, a group, a molecule other than a nucleic acid, a structure, or the like is linked. The nucleic acid may be a single-stranded nucleic acid or a double-stranded nucleic acid.

In one aspect, the nucleic acid may include mRNA. In one aspect, the nucleic acid-polypeptide conjugate may be an mRNA-polypeptide conjugate.

In one aspect, the nucleic acid may include cDNA. In one aspect, the nucleic acid-polypeptide conjugate may be a conjugate of an mRNA/cDNA double-stranded complex and a polypeptide (hereinafter, also referred to as an "mRNA-cDNA-polypeptide conjugate"). In one aspect, the nucleic acid-polypeptide conjugate may be a cDNA-polypeptide conjugate.

From the viewpoint of the purification yield, the length of the nucleic acid of the nucleic acid-polypeptide conjugate is preferably 30 bases or more, more preferably 70 bases or more, and still more preferably 100 bases or more. From the viewpoint of the formation efficiency of the nucleic acid-polypeptide conjugate, the length of the nucleic acid of the nucleic acid-polypeptide conjugate is preferably 2,000 bases or less, more preferably 1,000 bases or less, and still more preferably 500 bases or less. From such viewpoints, the length of the nucleic acid of the nucleic acid-polypeptide conjugate is preferably 30 bases to 2,000 bases, more preferably 70 bases to 1,000 bases, and still more preferably 100 bases to 500 bases. In a case where the nucleic acid is double-stranded, the "length of the nucleic acid" refers to the length of the nucleic acid of the single strand.

The nucleic acid used for the production of a conjugate may be, for example, a transcription product of a population of double-stranded DNA fragments produced by performing an overlap extension PCR using a set of random primers containing a random sequence. The random sequence is, for example, a repeating triplet sequence [NNK]ₘ (here, m is a positive integer, N's each independently represent A, T, G, or C, and K each independently represents T or G). It is possible to produce a peptide having a random amino acid sequence of an arbitrary length by setting the repetition number of the triplet [NNK] to an arbitrary number. The random sequence can also be produced by repeatedly linking an equal amount mixture of trimer oligonucleotides in which one type of codon is assigned to one type of amino acid. From the viewpoint of suppressing the appearance of the stop codon, the random sequence is preferably a trimer oligonucleotide rather than a repeating triplet [NNK].

The nucleic acid has a nucleotide sequence required to synthesize a polypeptide by a cell-free peptide synthesis system. The nucleotide sequence required for polypeptide synthesis includes not only a coding region but also, for example, a ribosome binding sequence. The nucleotide sequence of the template nucleic acid may include a stop codon or may not include a stop codon. Here, the stop codon means a codon in which the tRNA to be paired is not present in the reaction solution of the cell-free peptide synthesis system. From the viewpoint of the formation efficiency of a nucleic acid-polypeptide conjugate, it is preferable that the nucleotide sequence of the template nucleic acid does not contain a stop codon.

From the viewpoint of reducing steric hindrance caused by the nucleic acid to the action of the polypeptide, it is preferable that a nucleotide sequence encoding a spacer is present on the 3'-end side of the template nucleic acid. The spacer is, for example, 1 to 20 amino acid residues or peptide residues selected from glycine and serine.

The polypeptide of the nucleic acid-polypeptide conjugate is a polypeptide expressed from a nucleic acid, and the polypeptide is not limited in terms of a three-dimensional structure, an amino acid sequence, the number of amino acid residues, the type of amino acid, the nucleotide sequence encoding the polypeptide, and the like. In the present disclosure, a polypeptide refers to a molecule in which amino acids are linked by a peptide bond. The number of amino acid residues of the polypeptide is not limited, and the polypeptide is a term including a protein. The polypeptide includes a polypeptide in which an amino acid is post-translationally modified. Examples of the post-translational modification of an amino acid include phosphorylation, methylation, acetylation, glycosylation, lipidation, and the like.

In the present disclosure, the amino acid includes a natural amino acid, an unnatural amino acid, a modified amino acid, and a derivative thereof.

In the present disclosure, the natural amino acid refers to an amino acid that constitutes a general protein, and examples thereof include alanine (Ala, A), arginine (Arg, R), asparagine (Asn, N), aspartic acid (Asp, D), and cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V). The natural amino acid may be a natural product or an artificial product.

In the present disclosure, the unnatural amino acid refers to an amino acid other than the twenty types of amino acids described above, and includes a natural product or an artificial product. Examples of the unnatural amino acid include an amino acid having a haloacetyl group (for example, chloroacetylated amino acids such as chloroacetylated lysine or chloroacetylated diaminobutyric acid), an N-methyl amino acid (for example, N-methylalanine or N-methylphenylalanine), and the like.

Examples of the modified amino acid include a labeled amino acid, which is an amino acid bonded to a labeling compound, and the like. The labeling compound is a substance that can be detected by a biochemical, chemical, immunochemical, or electromagnetic detection method. Examples of the labeling compound include a coloring agent compound, a fluorescent substance, a chemiluminescent substance, a bioluminescent substance, an enzyme substrate, a coenzyme, an antigenic substance, a substance that binds to a specific protein, a magnetic substance, and the like. Examples of the labeled amino acid include, as classified according to function, a fluorescent-labeled amino acid, a photoresponsive amino acid, a photoswitch amino acid, a fluorescent probe amino acid, and the like.

The amino acid in the labeled amino acid and the labeling compound may be directly bonded to each other or may be bonded to each other through a spacer. Examples of the spacer include polyolefins such as polyethylene and polypropylene; polyethers such as polyoxyethylene, polyethylene glycol, and polyvinyl alcohol; polystyrene, polyvinyl chloride, polyester, polyamide, polyimide, polyurethane, polycarbonate, and the like.

Examples of the derivative of the natural amino acid, the unnatural amino acid, or the modified amino acid include hydroxy acids, mercapto acids, and carboxylic acids.

In one aspect, the polypeptide may be a polypeptide containing an unnatural amino acid. Examples of the polypeptide containing an unnatural amino acid include the polypeptide containing unnatural amino acids exemplified above.

In one aspect, the polypeptide may be a cyclic polypeptide containing an unnatural amino acid. Examples of the cyclic polypeptide containing an unnatural amino acid include a polypeptide containing, in one molecule, an amino acid having a thiol group (for example, cysteine) and an amino acid having a chloroacetyl group (for example, chloroacetyl diaminobutyric acid or chloroacetylated lysine). In such a polypeptide, the thiol group reacts with the chloroacetyl group to undergo cyclization, resulting in a cyclic polypeptide.

From the viewpoint of expressing the polypeptide activity, the number of amino acid residues of the polypeptide of the nucleic acid-polypeptide conjugate is preferably 6 or more. In addition, from the viewpoint of formation efficiency of the nucleic acid-polypeptide conjugate, the number of amino acid residues of the polypeptide of the nucleic acid-polypeptide conjugate is preferably 600 or less, more preferably 300 or less, and still more preferably 150 or less. From such viewpoints, the number of amino acid residues of the polypeptide of the nucleic acid-polypeptide conjugate is preferably 6 to 600, more preferably 6 to 300, and still more preferably 6 to 150.

In the nucleic acid-polypeptide conjugate, the nucleic acid and the polypeptide may be linked to each other via a linker. From the viewpoint that the polypeptide easily forms an appropriate higher-order structure and the viewpoint that the nucleic acid-polypeptide conjugate has high stability and thus the function of the polypeptide is easily evaluated, it is preferable that the nucleic acid and the polypeptide are linked via a linker. In the present disclosure, the linker is a structure that functions as a linking part that links a nucleic acid and a corresponding polypeptide.

Examples of the linker include a compound containing a puromycin-like compound and a single-stranded nucleic acid of 10 to 100 bases (hereinafter, this compound is also referred to as a "puromycin linker"). The puromycin linker bonds to a polypeptide via a puromycin-like compound. In addition, the single-stranded nucleic acid of the puromycin linker has a sequence (hereinafter, also referred to as a "complementary part") complementary to a part of the nucleic acid moiety (for example, the 3' end of mRNA) constituting the nucleic acid-polypeptide conjugate, and binds to the nucleic acid moiety at the complementary part. In this manner, a nucleic acid-polypeptide conjugate is formed.

In the puromycin linker, the puromycin-like compound refers to a compound having a chemical structural skeleton similar to the 3' end of aminoacyl tRNA, and having an ability to bond to the C-terminus of a synthesized polypeptide in a case where the synthesis of a protein is performed in a cell-free peptide synthesis system. The aminoacyl tRNA is a tRNA in which an amino acid is covalently bonded to the tRNA. Examples of the puromycin-like compound include, in addition to puromycin, 3'-N-aminoacyl puromycin amino nucleosides (PANS-amino acids, for example, PANS-Gly, PANS-Val, PANS-Ala, and the like); 3'-N-aminoacyl adenosine amino nucleosides (AANS-amino acids; for example, AANS-Gly, AANS-Val, AANS-Ala, and the like) linked by an amide bond formed by dehydration condensation of an amino group of 3-aminoadenosine and a carboxy group of an amino acid; a compound in which a nucleoside or a nucleoside and an amino acid are ester-bonded; and ribocytidyl puromycin, deoxycytidyl puromycin, deoxyuridyl puromycin, and the like.

In the puromycin linker, the complementary part indicates a site of a single-stranded nucleic acid that has a sequence complementary to a part of the nucleic acid in the nucleic acid-polypeptide conjugate, thereby binding to the nucleic acid. From the viewpoint of the formation efficiency of a nucleic acid-polypeptide conjugate, the complementary part is preferably bonded to the 3'-end side of the region of the nucleic acid, which encodes the polypeptide. In addition, from the viewpoint of suppressing the dissociation of the nucleic acid and the puromycin linker, it is preferable to use a 2'-O-methylated complementary part. In addition, from the viewpoint of suppressing the dissociation of the nucleic acid and the puromycin linker, it is preferable that the complementary part and the nucleic acid moiety form a covalent bond by introducing an ultraviolet crosslinking compound or the like. The number of bases in the complementary part is preferably 15 to 40 and more preferably 20 to 30.

The puromycin linker may contain a spacer between the puromycin-like compound and the complementary part. The spacer may have any structure capable of linking the puromycin-like compound and the complementary part, and examples thereof include a polynucleotide, polyalkylene (polyethylene or the like), polyalkylene glycol (polyethylene glycol or the like), peptide nucleic acid, polystyrene, a combination of these, and the like. In a case where the above-described structures are combined, they can be chemically linked by an appropriate linking group (-NH-, -CO-, -O-, -NHCO-, -CONH-, -NHNH-, -(CH₂)ₙ- (n is a positive integer), -S-, -SO-, or the like).

Examples of the puromycin linker include the linker described in WO2005/012902A.

The nucleic acid-polypeptide conjugate may or may not have a modification molecule. In the present disclosure, the modification molecule in the nucleic acid-polypeptide conjugate refers to a molecule added to the portion other than the nucleic acid encoding the polypeptide and the polypeptide in the nucleic acid-polypeptide conjugate. The modification molecule also includes the labeling compound in the above-described linker (puromycin linker and the like) and the above-described modified amino acid. Examples of the other modification molecules include molecules that impart binding specificity, such as biotin, azide, alkyne, and affinity tag; His tag, FLAG tag, and the like.

Hereinafter, each step of the production method of a nucleic acid display library of the present disclosure will be described.

### [Conjugate production step]

In the conjugate production method, the nucleic acid-polypeptide conjugate is produced by expressing a polypeptide from a nucleic acid and linking the nucleic acid to the polypeptide.

In one aspect, the conjugate production step is performed by a cell-free peptide synthesis system. The cell-free peptide synthesis system is a reaction system for polypeptide synthesis, and is a cell-free peptide synthesis system that uses components present in cells such as Escherichia coli without using cells such as Escherichia coli as they are, and there are a cell-free peptide synthesis system using a cell extract and a cell-free peptide synthesis system using a reconstituted reaction solution (reconstituted cell-free peptide synthesis system) obtained by purifying and reconstituting each component of a cell extract.

In systems using a cell extract, examples of the cell extract include an Escherichia coli extract, a wheat germ extract, a rabbit erythrocyte extract, and an insect cell extract.

The reconstituted cell-free peptide synthesis system can be constructed with ribosomal proteins, aminoacyl tRNA synthetase (ARS), ribosomal RNA, amino acids, GTP, ATP, translation initiation factor (IF), elongation factor (EF), termination factor (RF), ribosome recycling factor, and other factors required for cell-free peptide synthesis system, each of which has been purified.

In the conjugate production step, any known cell-free peptide synthesis system can be adopted. Examples of commercially available products of the cell-free peptide synthesis system include PUREfrex (GeneFrontier), PURExpress In Vitro Protein Synthesis Kit (New England BioLabs), S30 T7 High-Yield Protein Expression System (Promega), Human Cell-Free Protein Expression System (Takara Bio), Rapid Translation System (Roche), Expressway Cell-Free Expression System (Invitrogen), and the like.

In a case of producing a nucleic acid-polypeptide conjugate containing a puromycin linker as a linker, it is preferable that the nucleic acid to be subjected to the cell-free peptide synthesis system be a nucleic acid to which a puromycin linker is bonded at a 3' end.

In one aspect, the polypeptide to be expressed may be a polypeptide containing an unnatural amino acid. The details of the unnatural amino acid are as described above.

In one aspect, the polypeptide to be expressed is a polypeptide which contains an amino acid having a first functional group and an amino acid having a second functional group that is covalently bonded to the first functional group, in which 6 to 16 amino acids are interposed between the amino acid having the first functional group and the amino acid having the second functional group. In such a polypeptide, the first functional group and the second functional group can react with each other to be cyclized, and can be formed into a cyclic polypeptide.

In a case where the polypeptide to be expressed has the above-described form, the conjugate production step includes cyclizing the polypeptide of the nucleic acid-polypeptide conjugate by a covalent bond between the first functional group and the second functional group. The cyclization may be formed in the mRNA-polypeptide conjugate or may be formed in the mRNA-cDNA-polypeptide conjugate or the cDNA-polypeptide conjugate.

The first functional group and the second functional group may be the same type of functional group or different types of functional groups as long as the first functional group and the second functional group are covalently bonded to each other. Examples of the combination of the first functional group and the second functional group include a combination of a thiol group and a chloroacetyl group, a combination of a thiol group and a thiol group, and a combination of a carboxy group in a side chain and an amino group in a side chain.

Examples of the amino acid having a thiol group include cysteine.

Examples of the amino acid having a chloroacetyl group include chloroacetyl diaminobutyric acid and chloroacetylated lysine.

Examples of the amino acid having a carboxy group in the side chain include aspartic acid and glutamic acid.

Examples of the amino acid having an amino group in the side chain include lysine, asparagine, and glutamine.

In one aspect, the production method of a nucleic acid display library of the present disclosure may further include producing a conjugate of an mRNA/cDNA double-stranded complex and a polypeptide by reverse transcription of the mRNA. According to the present aspect, since the nucleic acid moiety is a double-stranded nucleic acid, a nucleic acid display library in which various effects due to the formation of the tertiary structure of the mRNA are eliminated can be obtained, which is more preferable.

### [Separation step]

In the separation step, the nucleic acid-polypeptide conjugate is separated from a mixture of a polypeptide not linked to the nucleic acid and the nucleic acid-polypeptide conjugate, independently of a nucleotide sequence of the nucleic acid and the modification molecule.

It is noted that in the present disclosure, "separation of a nucleic acid-polypeptide conjugate from a mixture of a polypeptide not linked to the nucleic acid and the nucleic acid-polypeptide conjugate, independently of a nucleotide sequence of the nucleic acid and the modification molecule" means separating between the polypeptide not linked to the nucleic acid and the nucleic acid-polypeptide conjugate, and does not necessarily mean that only the nucleic acid-polypeptide conjugate is isolated. For example, the obtained nucleic acid-polypeptide conjugate and other substances such as an unlinked nucleic acid may be intermixed.

In one aspect, the separation of the nucleic acid-polypeptide conjugate is preferably performed by the following method.
(1) The mixture obtained in the conjugate production step is brought into contact with a solid-phase carrier capable of binding to a nucleic acid of the nucleic acid-polypeptide conjugate. Hereinafter, this method is also referred to as a "solid-phase adsorption method".
(2) A solution capable of insolubilizing a nucleic acid is added to the mixture obtained in the conjugate production step. Hereinafter, this method will also be referred to as an "insolubilization method".

The methods (1) and (2) are particularly simple examples of the separation method. Among these, from the viewpoints of purification yield, removal efficiency of impurities, and processing speed, a solid-phase adsorption method is preferable.

### -Solid-phase adsorption method-

In the solid-phase adsorption method, the mixture is brought into contact with a solid-phase carrier capable of binding to the nucleic acid of the nucleic acid-polypeptide conjugate (hereinafter, also referred to as an "adsorption step").

In one aspect, the production method of a nucleic acid display library of the present disclosure may further include eluting the nucleic acid-polypeptide conjugate bound to the solid-phase carrier with water or an aqueous solution (hereinafter, also referred to as an "elution step").

The surface material of the solid phase used in the solid-phase adsorption method may be any material as long as the nucleic acid is adsorbed. The nucleic acid may be adsorbed to the carrier by any mode such as a covalent bond, chemical adsorption, physical adsorption, an electrical interaction, a hydrophobic interaction, a van der Waals force, or a hydrogen bond. From the viewpoint of reducing the incorporation of impurities into the eluted solution, the surface material of the solid phase is preferably a material that enables the elution of the nucleic acid-polypeptide conjugate with water.

Examples of the nucleic acid adsorption mechanism that allows the nucleic acid-polypeptide conjugate to be eluted with water include mechanisms using a material that interacts chaotropically with the nucleic acid. Examples of the surface material that interacts chaotropically with a nucleic acid include silica, glass, polysaccharides (cellulose, dextran, agarose, and the like), carboxy group-containing polymers, hydroxy group-containing polymers, hydroxyapatites, and the like.

The form of the solid phase used in the solid-phase adsorption method is not particularly limited, and may be, for example, a column, a resin, or magnetic beads. From the viewpoint of the purification yield, the resin and the magnetic beads are preferable, and from the viewpoint of the processing speed, the magnetic beads are more preferable.

In one aspect, the magnetic beads are preferably magnetic beads coated with silica and magnetic beads coated with a carboxy group.

The adsorption step may be performed by bringing, into contact with a solid-phase carrier, a mixture obtained by mixing a solution capable of insolubilizing a nucleic acid with the mixture obtained in the conjugate production step.

Examples of the solution capable of insolubilizing a nucleic acid include a solution containing a component that removes hydration water from nucleic acids. Examples of the component that removes hydration water from nucleic acids include a chaotropic salt, a water-soluble organic solvent, a water-soluble polymer, and the like. The component that removes hydration water from nucleic acids may be used alone or in combination of two or more types thereof.

Examples of the chaotropic salt include sodium chloride, potassium chloride, ammonium chloride, sodium bromide, potassium bromide, calcium bromide, ammonium bromide, sodium iodide, potassium iodide, sodium perchlorate, guanidine hydrochloride, guanidine thiocyanate, guanidine sulfate, guanidine isothiocyanate, sodium isothiocyanate, urea, sodium thiocyanate, potassium thiocyanate, ammonium isothiocyanate, and the like. The chaotropic salt may be used alone or in combination of two or more types thereof.

Examples of the water-soluble organic solvent include methanol, ethanol, isopropyl alcohol, n-propanol, n-butanol, 2-butanol, dimethyl sulfoxide, and the like. The water-soluble organic solvent may be used alone or in combination of two or more types thereof.

Examples of the water-soluble polymer include polyethylene glycol, polysaccharides, and the like. The water-soluble polymer may be used alone or in combination of two or more types thereof.

The concentration of the chaotropic salt in the mixture in the adsorption step may be appropriately determined within a range in which the nucleic acid can be adsorbed to the solid-phase carrier.

The elution step may be performed by bringing water or an aqueous solution into contact with the solid-phase carrier on which the nucleic acid-polypeptide conjugate has been adsorbed.

Examples of the aqueous solution for elution include an aqueous solution that does not contain the above-described components that remove hydration water from nucleic acids, or contains the component at a low concentration.

The total concentration of the dissolved substances in the aqueous solution for elution may be appropriately determined within a range in which the nucleic acid can be eluted, but it is preferable to be lower since the influence on the next step is small. For example, the total concentration of the dissolved substances in the aqueous solution may be 0.2 M or less, 0.1 M or less, or 0.05 M or less. The total concentration of the dissolved substances in the aqueous solution may be 0.0001 M or more, 0.001 M or more, or 0.01 M or more. Therefore, the total concentration of the dissolved substances in the aqueous solution may be 0.0001 M to 0.2 M, 0.001 M to 0.1 M, or 0.01 M to 0.05 M. The dissolved substance is any substance to be dissolved in an aqueous solution, and examples thereof include acids, alkalis, salts, surfactants, and the like.

In one aspect, after the mixture is brought into contact with the solid-phase carrier at the first pH in the adsorption step, the nucleic acid-polypeptide conjugate may be eluted with an aqueous solution having the second pH in the elution step. The first pH and the second pH are different pHs.

The first pH is preferably 1.0 to 6.5 and more preferably 3.0 to 6.5.

The second pH is preferably 7.5 to 10.0 and more preferably 8.0 to 9.0.

It is preferable to wash the solid-phase carrier between the adsorption step and the elution step. The washing can be performed using a solution in which the adsorption of the nucleic acid-polypeptide conjugate to the solid-phase carrier can be maintained, and for example, the above-described solution can be used as a solution capable of insolubilizing the nucleic acid. In addition, washing may be performed using an aqueous solution of alcohol (70% ethanol or the like).

### -Insolubilization method-

In the insolubilization method, a solution capable of insolubilizing a nucleic acid is added to the mixture. The solution capable of insolubilizing a nucleic acid is as described above. The composition of the solution that insolubilizes the nucleic acid may be appropriately determined as long as the nucleic acid can be precipitated. In addition, the insolubilization may be promoted by adding the solution that insolubilizes the nucleic acid and then performing cooling. The cooling range is preferably 4°C or lower and more preferably - 20°C or lower.

In a case where the mRNA is reverse transcribed to produce an mRNA-cDNA-polypeptide conjugate, the separation step may be performed on the mRNA-polypeptide conjugate before reverse transcription or may be performed on the mRNA-cDNA-polypeptide conjugate after reverse transcription. It is preferable that the separation step be performed on the mRNA-cDNA-polypeptide conjugate after the reverse transcription, since the total amount of nucleic acids is increased and the recovery efficiency during the purification can be expected to be improved.

The production method of a nucleic acid display library may include other steps in addition to the conjugate production step and the separation step. For example, the method may include a step of producing a cDNA-polypeptide conjugate by causing a ribonuclease to act on an mRNA-cDNA-polypeptide conjugate, a step of modifying or labeling a polypeptide in a nucleic acid-polypeptide conjugate, and a step of separating and/or purifying the nucleic acid-polypeptide conjugate depending on a nucleotide sequence of the nucleic acid, an amino acid sequence of the polypeptide, or a modification molecule.

In a case of producing a cDNA-polypeptide conjugate from an mRNA-cDNA-polypeptide conjugate, the separation step may be performed on the mRNA-cDNA-polypeptide conjugate or may be performed on the cDNA-polypeptide conjugate.

### <Screening method>

A screening method of the present disclosure includes producing a nucleic acid display library by the above-described production method of a nucleic acid display library of the present disclosure, and selecting a nucleic acid-polypeptide conjugate having a target activity from the nucleic acid display library and identifying a nucleotide sequence of a nucleic acid of the selected nucleic acid-polypeptide conjugate.

The "target activity" is, for example, binding to a target substance. The target substance is a term including any chemical substance exhibiting physiological activity, and also including a compound, a group, a molecule, a protein, a nucleic acid, a lipid, a saccharide, a complex of these, and the like. The target substance is, for example, a receptor, a transcription factor, an enzyme, a coenzyme, a regulating factor, an antibody, an antigen, DNA, RNA, an exosome, a cell, a tissue, a fragment thereof, a complex thereof, and a modification group thereof.

In one aspect, the screening method of the present disclosure includes bringing a nucleic acid display library into contact with a target substance and performing incubation. For example, the nucleic acid display library is brought into contact with the target substance in a buffer solution, and incubated with the pH, temperature of the buffer solution, and contact time adjusted. A target substance may be immobilized on a solid-phase carrier, and then a nucleic acid display library may be brought into contact with the immobilized target substance. The solid-phase carrier is not limited as long as it is a carrier on which a target substance can be immobilized, and examples thereof include a microtiter plate, a substrate, a bead, a magnetic bead, a nitrocellulose membrane, a nylon membrane, a PVDF membrane, and the like. The target substance is immobilized on a solid-phase carrier by a known technique.

After the contact described above, the nucleic acid-polypeptide conjugate bound to a target substance is extracted, and the nucleotide sequence of the nucleic acid of the extracted nucleic acid-polypeptide conjugate is identified. Identification of the nucleotide sequence can be carried out using a nucleic acid amplification system and a sequencer.

The nucleic acid amplification system refers to a system that amplifies a nucleic acid using a nucleic acid as a template. The nucleic acid amplification reaction of the nucleic acid amplification system can be any one of polymerase chain reaction (PCR), ligase chain reaction (LCR), transcription mediated amplification (TMA), nucleic acid sequence-based amplification (NASBA), or the like.

In the present disclosure, the term "sequencer" includes a first generation sequencer (a capillary sequencer), a second generation sequencer (a next generation sequencer), a third generation sequencer, a fourth generation sequencer, and a sequencer to be developed in the future. The sequencer may be a capillary sequencer, may be a next generation sequencer, or may be another sequencer. The sequencer is preferably a next generation sequencer from the viewpoints of the speed of analysis, the large number of specimens that can be processed at one time, and the like. The next generation sequencer (NGS) refers to a sequencer that is classified by being contrasted with a capillary sequencer (called a first generation sequencer) using the Sanger method. At present, the most popular next generation sequencer is a sequencer of which the principle is to capture fluorescence or luminescence linked to a complementary strand synthesis by DNA polymerase or a complementary strand binding by DNA ligase and determine the nucleotide sequence. Specific examples thereof include MiSeq (Illumina, Inc., MiSeq is a registered trademark), HiSeq 2000 (Illumina, Inc., HiSeq is a registered trademark), Roche 454 (Roche Diagnostics GmbH), and the like.

### <Production method of polypeptide>

The production method for a polypeptide of the present disclosure includes acquiring a polypeptide having the target activity based on a nucleotide sequence identified by the above-described screening method of the present disclosure. The screening method and the identification method of a nucleotide sequence are as described above. The polypeptide can be acquired using a known polypeptide synthesis system based on the identified nucleotide sequence. Examples of the polypeptide synthesis system include a chemical synthesis system and a genetic engineering synthesis system, in addition to the above-described cell-free peptide synthesis system. From the viewpoint of purity, chemical synthesis system is preferable.

The production method of a nucleic acid display library, the screening method, and the production method of a polypeptide of the present disclosure can be widely used for the development of a therapeutic drug, the development of a diagnostic drug, the development of a reagent for research, the production of a pharmaceutical product, the production of a biomaterial, and the like.

### Examples

Hereinafter, the embodiment of the present disclosure will be more specifically described with reference to Examples, but the embodiment of the present disclosure is not limited to the following Examples.

### <Example 1>

### Construction of conditions for separation step

After the conjugate production step and the separation step, it was confirmed by the following method that the unlinked polypeptide was removed.

The sequence set forth in SEQ ID NO: 1 was used as a nucleic acid sequence for evaluation. In this library, the cell-free peptide synthesis (translation) initiates from the initiation codon (ATG) at the positions 86 to 88 from the 5' end, the bases at the positions 89 to 106 are sequences that increase the amount of cell-free peptide synthesis, the bases at positions 107 to 139 are sequences that encode HiBiT tag (Promega), and a base group (NNNTGT(NNN)₁₂TAGNNN (NNN is the trimer oligonucleotide described in Table 1 and N's each independently represent A, T, G, or C)) at the position 161 and thereafter is a random sequence, and the base group located on the 3'-end side of the random sequence is a linkage part of the puromycin linker and a stop codon. The triplet TAG in the present random sequence corresponds to a codon of chloroacetylated lysine, which is an unnatural amino acid. Therefore, in the polypeptide encoded by the random sequence, a thiol group of cysteine and a chloroacetyl group of chloroacetylated lysine spontaneously form a thioether bond to form a cyclic polypeptide. A base group at or before the 85th position from the 5'-end is a sequence necessary for transcription and initiation of cell-free peptide synthesis, such as a T7 promoter sequence or a Shine-Dalgarno sequence.

SEQ ID NO: 1:
GAAATTAATACGACTCACTATAGGGAGACCACAACGGTTTCCCTCTAGAAATAAT
TTTGTTTAACTTTAAGAAGGAGATATACATATGGTTAAGAAAACAAAAACAGTAA
GTGGATGGCGATTATTCAAGAAGATTAGCGAAGAAGGAGATATACATATGNNNT
GT(NNN)12TAGNNNGGTGGCTCTGGCGGTAGCAGGACGGGGGCGGCGGGGGGTA
AATAAATAAGCTTGAGTAT

**[Table 1]**

| Amino acid (one-letter notation) | Assigned codon | Amino acid (one-letter notation) | Assigned codon |
|---|---|---|---|
| A | GCT | N | AAC |
| I | ATC | Q | CAG |
| L | CTG | R | CGT |
| V | GTT | H | CAT |
| F | TTC | K | AAA |
| W | TGG | D | GAC |
| Y | TAC | E | GAA |
| S | TCT | G | GGT |
| T | ACT | P | CCG |

The library set forth in SEQ ID NO: 1 was produced by performing overlap extension PCR. Specifically, a library was produced by mixing three types of DNAs, the DNA set forth in SEQ ID NO: 2, the DNA set forth in SEQ ID NO: 3, and the DNA set forth in SEQ ID NO: 4, such that the concentrations thereof were 3 µmol/L, 1 µmol/L, and 1 µmol/L, repeatedly performing, after 98°C/30 seconds, three steps of 98°C/10 second, 60°C/10 seconds, and 72°C/10 seconds for 7 cycles in the presence of Platinumtm SuperFi II DNA Polymerase (Thermo, 12361010), and finally performing the treatment of 72°C/5 minutes, thereby linking the three DNAs. The produced library was purified and diluted to 10 ng/µL. In the DNA set forth in SEQ ID NO: 4, the trimer oligonucleotide represented by NNN is an equal amount mixture of trimer oligonucleotides corresponding to 18 types of codons shown in Table 1, in which one type of codon is assigned to one type of amino acid.
SEQ ID NO: 2: 
GAAATTAATACGACTCACTATAGGGAGACCACAACGGTTTCCCTCTAGAAATAAT
TTTGTTTAACTTTAAGAAGGAGATATACATATGGTTAAGAAAACAAAAACAGTAA
GTGGATGGCGATTATTCAAGAAGATTAGCGAAGAAGGAGATATACATATG
SEQ ID NO: 3: 
ATACTCAAGCTTATTTATTTATTACCCCCGCCGCCCCCGTCCTGCTACCGCCAG
AACCACC
SEQ ID NO: 4:

AAGAAGGAGATATACATATGNNNTGT(NNN)12TAGNNNGGCGGTTCTGGCGGTAG
C

The produced library (SEQ ID NO: 1) was reacted at 37°C for 30 minutes in the presence of T7 RNA Polymerase (TaKaRa, 2540A) to produce a library transcript. This mRNA fragment was purified and diluted to 10 µM.

Next, the library transcript (final concentration: 5.0 µM) and the puromycin linker set forth in SEQ ID NO: 5 (final concentration: 10 µM) were mixed in a TBS buffer (1.25 mM Tris, 25 mM NaCl, pH: 7.5), then heated at 95°C for 5 minutes, and irradiated on ice with 10 J of UV (365 nm) to produce a complex of the library transcript and the puromycin linker. As a comparative target, an example in which water was added instead of the puromycin linker was prepared and used for evaluating the separation efficiency of the polypeptide. In this comparative example, since the nucleic acid and the polypeptide are not linked in principle, the residual amount of the polypeptide in this comparative example can be used as an index for measuring the separation efficiency.

SEQ ID NO: 5: (PsoralenC6)-UACCCCCCGCCGCCCCCCGUCCU~(Sp18)-(Sp18)-(Sp18)-(Sp18)-CC-(Puro) (See the structures of PsoralenC6, Sp18, and Puro in the following figure, and the nucleotides between (PsoralenC6) and (Sp18) are 2'-O-methyl-modified RNAs, and the other nucleotides are unmodified DNAs.)

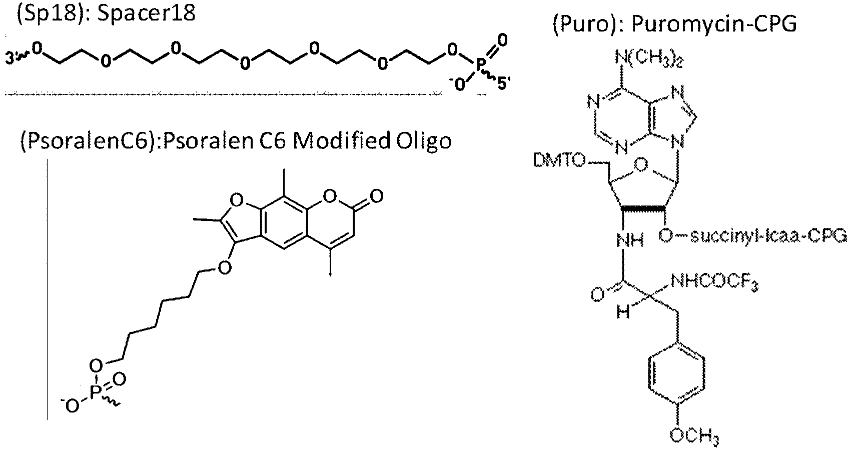

To perform cell-free peptide synthesis of chloroacetylated lysine which is an unnatural amino acid, a tRNA in which the anticodon was CUA and that pairs with the UAG codon in the mRNA was prepared by transcribing the DNA of SEQ ID NO: 34. The tRNA was aminoacylated with an N-chloroacetylated lysine 5'-phospho-2'-deoxyribocytidylylriboadenosine (pdCpA) ester. This aminoacyl tRNA is referred to as Aminoacyl tRNA (1).

SEQ ID NO: 34:
GTTGTAAAACGACGGCCAGTGCCAAGCTTGGGCTAATACGACTCACTATAGGGA

GAGTAGTTCAATGGTAGAACGTCGGTCTCTAAAACCGAGCGTTGAGGGTTCGATT
CCTTTCTCTCCCAC

A cell-free peptide synthesis was performed by reacting a complex of the library transcript and the puromycin linker in a cell-free peptide synthesis solution containing PUREfrex 2.0 (GeneFrontier, PF201-0.25-5) and Aminoacyl tRNA (1) (6 µL of the complex/20 µL of the reaction volume, a final concentration of Aminoacyl tRNA (1) of 0.5 µg/µL) at 37°C for 60 minutes, thereby producing a nucleic acid (mRNA)-polypeptide conjugate.

The unlinked polypeptide was separated from the cell-free peptide synthetic product using a column or magnetic beads. An example in which the present separation step was not performed as a comparative target was also prepared.

The separation step using a column was performed using an RNeasy MinElute Cleanup Kit (QIAGEN, 74204, silica gel membrane). First, 14 µL of the cell-free peptide synthetic product, 50 µL of Buffer RLT, and 70 µL of ethanol were mixed, and then the mixture was passed through a spin column attached to the kit to bind the nucleic acid-polypeptide conjugate to the silica gel membrane in the spin column. After washing with 500 µL of Buffer RPE and 500 µL of 80% ethanol, the nucleic acid-polypeptide conjugate was eluted using 14 µL of water.

The separation step using magnetic beads was performed using RNAClean XP (Beckman Coulter, A63987, carboxy group-containing polymer-coated magnetic beads). First, 14 µL of the cell-free peptide synthetic product and 25 µL of RNAClean XP beads were mixed and shaken for 5 minutes to bind the nucleic acid-polypeptide conjugate to the RNAClean XP beads. Next, after being left to stand on a magnet rack until the magnetic beads settled, the supernatant was removed, the magnetic beads were washed five times with 200 µL of 70% ethanol, and 14 µL of water was added thereto and left to stand for 5 minutes to elute the nucleic acid-polypeptide conjugate. After allowing the magnetic beads to stand on the magnet rack until the magnetic beads settled, the eluate supernatant was collected.

The 10.6 µL of the eluted product or the 10.6 µL of the cell-free peptide biosynthetic product was mixed with the DNA of SEQ ID NO: 6 (final concentration of 10 µM) in the presence of ReverTra Ace (TOYOBO, TRT-101) (reaction volume of 16 µL), and reacted at 42°C for 30 minutes to perform reverse transcription, thereby producing a nucleic acid (cDNA and mRNA)-polypeptide conjugate.
SEQ ID NO: 6: GCTACCGCCAGAACCACC

For the reverse transcription product or the unlinked polypeptide-removal product, the polypeptide concentration and the nucleic acid concentration were quantified for each of the type of the separation step of separating the unlinked polypeptide and whether or not it was performed, and the presence or absence of the puromycin linker necessary for linking the polypeptide to the nucleic acid. The removal rate of the unlinked polypeptide was calculated from the concentration of the polypeptide under the condition in which the puromycin linker was not added, and the yield of the nucleic acid-polypeptide conjugate was calculated from the concentration of the nucleic acid under the condition in which the puromycin linker was added.

The polypeptide concentration was quantified using a HiBiT tag. Specifically, the measurement was performed using the Nano Glo HiBiT Lytic Detection System (Promega, N3040) and a chemically synthesized polypeptide for a calibration curve with a known concentration (SEQ ID NO: 7) according to the standard protocol of the Nano Glo HiBiT Lytic Detection System. The quantification results are shown in Table 2.
SEQ ID NO: 7: EQKLISEEDLGGSVSGWRLFKKIS

The nucleic acid concentration was quantified by a qPCR method using the primers of SEQ ID NO: 8 and SEQ ID NO: 9 and a DNA for a calibration curve (SEQ ID NO: 10) with a known concentration.
SEQ ID NO: 8: GGAGATATACATATGGTTAAGAAAACAAAAAC
SEQ ID NO: 9: CTGCTACCGCCAGAACCACC
SEQ ID NO: 10:

GCTACCGCCAGAACCACCTGCCTAACGATAGTTATCACCACGCGGTTCACATGCT
GTTTTTGTTTCTTAACCATATGTATATCTCCTTCTTAAAGTTAAACAAAATTATTT
CTAGAGGGAAACCGTTGTGGTCTCCC

**[Table 2]**

| No. | Puromycin linker | Separation step | Polypeptide concentration (nM) | Nucleic acid concentration (nM) |
|---|---|---|---|---|
| 1 | Presence | Column | 190 | 470 |
| 2 | Presence | Magnetic beads | 180 | 410 |
| 3 | Presence | Absence | 6,300 | 640 |
| 4 | Absence | Column | 60 | 640 |
| 5 | Absence | Magnetic beads | 60 | 450 |
| 6 | Absence | Absence | 6,800 | 720 |

Nos. 4 to 6 are conditions in which the puromycin linker is not added, and all polypeptides detected under these conditions are unlinked polypeptides. As compared with No. 6 in which the separation step was not performed, in No. 4 and No. 5, the polypeptide concentration was 1/100 or less due to the separation step, it was indicated that 99% or more of the unlinked polypeptides could be removed by the production method of a display library of the present disclosure.

Nos. 1 to 3 are conditions in which puromycin linker is added, and the detected polypeptide concentration is the sum of the unlinked polypeptide and the nucleic acid-polypeptide conjugate. Therefore, the polypeptide concentrations of No. 1 and No. 2 were higher than the polypeptide concentrations of No. 4 and No. 5. The polypeptide concentrations of No. 1 and No. 2 were reduced to about 3% of that of No. 3. On the other hand, the nucleic acid concentrations of No. 1 and No. 2 were about 73% and about 64% with respect to No. 3, and the loss of the nucleic acid-polypeptide conjugate in the separation step was as low as about 27% and about 36%. This indicates that the practicality of the present separation method is high.

### <Example 2>

### Production of nucleic acid-polypeptide conjugate using various cell-free peptide synthesis systems

A nucleic acid-polypeptide conjugate was produced using a PUREexpress In Vitro Protein Synthesis Kit (NEB, E6800) and a S30 T7 High-Yield Protein Expression System (Promega, L1110), in addition to the PUREfrex described in Example 1. Steps other than the cell-free peptide synthesis are the same as in Example 1.

The cell-free peptide synthesis step was performed according to the following procedure.

A cell-free peptide synthesis was performed by reacting a complex of the library transcript and the puromycin linker at 37°C for 60 minutes in a cell-free peptide synthesis solution containing a PUREexpress In Vitro Protein Synthesis Kit (6 µL of complex/reaction volume of 20 µL, final concentration of Aminoacyl tRNA (1) of 0.5 µg/µL) or in an S30 T7 High-Yield Protein Expression System (4.8 µL of complex/reaction volume of 20 µL, final concentration of Aminoacyl tRNA (1) of 0.5 µg/µL), thereby producing a nucleic acid (mRNA)-polypeptide conjugate.

The quantification results are shown in Table 3. From the results of Nos. 7 and 8, it was shown that even in a case where the PURExpress In Vitro Protein Synthesis Kit was used, the nucleic acid-polypeptide conjugate could be produced and the polypeptide and the nucleic acid-polypeptide conjugate could be separated. In addition, in a case of using the S30 T7 High-Yield Protein Expression System, it was difficult to quantify the nucleic acid concentration without a separation step due to the influence of the component of the system, but from the results of Nos. 9 and 10, it was shown that a nucleic acid-polypeptide conjugate could be produced and the polypeptide and the nucleic acid-polypeptide conjugate could be separated.

**[Table 3]**

| No. | Puromycin linker | Cell-free peptide synthesis system | Separation step | Polypeptide concentration (nM) | Nucleic acid concentration (nM) |
|---|---|---|---|---|---|
| 1 | Presence | PUREfrex | Column | 190 | 470 |
| 3 | Presence | PUREfrex | Absence | 6300 | 640 |
| 7 | Presence | PURExpress | Column | 150 | 460 |
| 8 | Presence | PURExpress | Absence | 4900 | 630 |
| 9 | Presence | S30 | Column | 72 | 120 |
| 10 | Presence | S30 | Absence | 370 | Not determined |

### <Example 3>

### Implementation of mRNA display method

The polypeptide that binds to the FGFR1c protein was recovered from the random library using the production method of a display library of the present disclosure.

The sequences set forth in SEQ ID NOs: 11 to 21 were used as a library of the mRNA display method. In this library, the cell-free peptide synthesis initiates from the initiation codon (ATG) at the positions 86 to 88 from the 5' end, a base group (NNNTGT(NNN)_{6 to 16}TAGNNN (NNN is a trimer oligonucleotide, where N's each independently represent A, T, G, or C)) at the position 107 and thereafter is a random sequence, and a base group located on the 3'-end side of the random sequence is a linkage part of a puromycin linker and a stop codon. A base group at or before the 85th position from the 5'-end is a sequence necessary for transcription and initiation of cell-free peptide synthesis, such as a T7 promoter sequence or a Shine-Dalgarno sequence. The triplet TAG in this random sequence corresponds to a codon of chloroacetylated lysine. Therefore, in the polypeptide encoded by the random sequence, a thiol group of cysteine and a chloroacetyl group of chloroacetylated lysine spontaneously form a thioether bond to form a cyclic polypeptide. In the nucleic acids having the sequences set forth in SEQ ID NOs: 11 to 21, the trimer oligonucleotide represented by NNN is an equal amount mixture of trimer nucleotides corresponding to 18 types of codons shown in Table 1, in which one type of codon is assigned to one type of amino acid. Regarding the (NNN)_{6 to 16} part, 11 types of libraries having different repetition numbers of 6 to 16 were individually prepared, mixed in equal amount, and mRNA display was performed. 
SEQ ID NOs: 11 to 21:
GAAATTAATACGACTCACTATAGGGAGACCACAACGGTTTCCCTCTAGAAATAAT
TTTGTTTAACTTTAAGAAGGAGATATACATATGGTTAAGAAAACAAAAACANNNT
GT(NNN)6 to
 ₁₆TAGNNNGGTGGCTCTGGCGGTAGCAGGACGGGGGGCGGCGGGGGGTAAATAAA TAAGCTTGAGTAT (NNN is a trimer oligonucleotide, where N's each independently represent A, T, G, or C.)
(The repetition number of (NNN) is 6 in SEQ ID NO: 11, 7 in SEQ ID NO: 12, 8 in SEQ ID NO: 13, 9 in SEQ ID NO: 14, 10 in SEQ ID NO: 15, 11 in SEQ ID NO: 16, 12 in SEQ ID NO: 17, 13 in SEQ ID NO: 18, 14 in SEQ ID NO: 19, 15 in SEQ ID NO: 20, and 16 in SEQ ID NO: 21.)

The libraries set forth in SEQ ID NOs: 11 to 21 were produced by performing overlap extension PCR. Specifically, a library was produced by mixing three types of DNAs, the DNA set forth in SEQ ID NO: 22, the DNA set forth in SEQ ID NO: 3, and the DNA set forth in any of SEQ ID NOs: 23 to 33, such that the concentrations thereof were 3 µmol/L, 1 µmol/L, and 1 µmol/L, repeatedly performing, after 98°C/30 seconds, three steps of 98°C/10 seconds, 60°C/10 seconds, and 72°C/10 seconds for 7 cycles in the presence of Platinumtm SuperFi II DNA Polymerase (Thermo, 12361010), and finally performing the treatment of 72°C/5 minutes, thereby linking the three DNAs. The produced library was purified and diluted to 10 ng/µL. In the DNA set forth in SEQ ID NOs: 23 to 33, the trimer oligonucleotide represented by NNN is an equal amount mixture of trimer nucleotides corresponding to 18 types of codons shown in Table 1, in which one type of codon is assigned to one type of amino acid.
SEQ ID NO: 22: 
GAAATTAATACGACTCACTATAGGGAGACCACAACGGTTTCCCTCTAGAAATAAT
TTTGTTTAACTTTAAGAAGGAGATATACATATGGTTAAAAAACAAAAAC
SEQ ID NO: 23: 
AAGAAGGAGATATACATATGGTTAAAAAAACAAAAACANNNTGT(NNN)6TAGNN
NGGCGGTTCTGGCGGTAGC
SEQ ID NO: 24: 
AAGAAGGAGATATACATATGGTTAAAAAAACAAAAACANNNTGT(NNN)7TAGNN
NGGCGGTTCTGGCGGTAGC
SEQ ID NO: 25: 
AAGAAGGAGATATACATATGGTTAAAAAACAAAAACANNNTGT(NNN)8TAGNN
NGGCGGTTCTGGCGGTAGC
SEQ ID NO: 26: 
AAGAAGGAGATATACATATGGTTAAAAAAACAAAAACANNNTGT(NNN)9TAGNN
NGGCGGTTCTGGCGGTAGC
SEQ ID NO: 27: 
AAGAAGGAGATATACATATGGTTAAAAAAACAAAAACANNNTGT(NNN)10TAGNN
NGGCGGTTCTGGCGGTAGC
SEQ ID NO: 28: 
AAGAAGGAGATATACATATGGTTAAAAAACAAAAACANNNTGT(NNN)11TAGNN
NGGCGGTTCTGGCGGTAGC
SEQ ID NO: 29: 
AAGAAGGAGATATACATATGGTTAAAAAAACAAAAACANNNTGT(NNN)12TAGNN
NGGCGGTTCTGGCGGTAGC
SEQ ID NO: 30: 
AAGAAGGAGATATACATATGGTTAAAAAACAAAAACANNNTGT(NNN)13TAGNN
NGGCGGTTCTGGCGGTAGC
SEQ ID NO: 31: 
AAGAAGGAGATATACATATGGTTAAAAAAACAAAAACANNNTGT(NNN)14TAGNN
NGGCGGTTCTGGCGGTAGC
SEQ ID NO: 32: 
AAGAAGGAGATATACATATGGTTAAAAAACAAAAACANNNTGT(NNN)15TAGNN
NGGCGGTTCTGGCGGTAGC
SEQ ID NO: 33:

AAGAAGGAGATATACATATGGTTAAAAAACAAAAACANNNTGT(NNN)16TAGNN
NGGCGGTTCTGGCGGTAGC

Using Recombinant Human FGFR1 alpha (IIIc) Fc Chimera Protein, CF (R&D systems, 658-FR-050) as an FGFR protein, immobilization of the FGFR protein (FGFR protein concentration during immobilization: 1 µg/µL) was performed on magnetic beads (NHS Mag Sepharose, Cytiva, 28951380) according to a protocol designated by the manufacturer (Cytiva).

The produced libraries (SEQ ID NOs: 11 to 21) were reacted at 37°C for 30 minutes in the presence of T7 RNA Polymerase (TaKaRa, 2540A) to produce library transcripts. This mRNA fragment was purified and diluted to 10 µM.

Next, the library transcript (final concentration: 5 µM) and the puromycin linker set forth in SEQ ID NO: 5 (final concentration: 10 µM) were mixed in a TBS buffer (1.25 mM Tris, 25 mM NaCl, pH: 7.5), then heated at 95°C for 5 minutes, and irradiated on ice with 10 J of UV (365 nm) to produce a complex of the library transcript and the puromycin linker.

A nucleic acid (mRNA)-polypeptide conjugate was produced by performing a cell-free peptide synthesis reaction of a complex of the library transcript and the puromycin linker at 37°C for 60 minutes in a cell-free peptide synthesis solution containing PUREfrex (GeneFrontier) and an aminoacyl tRNA (6 µL of the complex/20 µL of the reaction volume, a final concentration of Aminoacyl tRNA (1) of 0.5 µg/µL).

Next, a separation step of unlinked polypeptides was performed. An example in which the present separation step was not performed as a comparative target was also prepared. In the separation step, first, 20 µL of the cell-free peptide synthetic product was mixed with 36 µL of RNAClean XP (Beckman Coulter, A63987) and shaken for 5 minutes to bind the nucleic acid-polypeptide conjugate to the RNAClean XP beads. Next, after being left to stand on a magnet rack until the magnetic beads settled, the supernatant was removed, the magnetic beads were washed five times with 200 µL of 70% ethanol, and 15 µL of water was added thereto and left to stand for 5 minutes to elute the nucleic acid-polypeptide conjugate. After allowing the magnetic beads to stand on the magnet rack until the magnetic beads settled, the eluate supernatant was collected.

15 µL of the elution product and the DNA (final concentration of 10 µM) of SEQ ID NO: 6 were mixed in the presence of ReverTra Ace (TOYOBO, TRT-101) (reaction volume of 37.5 µL) and reacted at 37°C for 30 minutes to perform reverse transcription and to produce a nucleic acid (cDNA and mRNA)-polypeptide conjugate.

Next, a selection step was performed. Specifically, the unlinked polypeptide removal product or the reverse transcription product was mixed with 10 µL of the FGFR immobilized on magnetic beads in a TBS buffer (20 mM Tris, 150 mM NaCl, 0.1% BSA, 0.05% Tween 20, pH 7.4) (reaction volume of 100 µL), and reacted at room temperature for 45 minutes, and then the magnetic beads were washed three times with 100 µL of the TBS buffer to extract a nucleic acid-polypeptide conjugate bound to the FGFR protein.

The amount of nucleic acid before and after the selection step were quantified by a qPCR method using the primers of SEQ ID NO: 8 and SEQ ID NO: 9 and the DNA for a calibration curve (SEQ ID NO: 10) having a known concentration, and the recovery rate of the polypeptide in the selection step was calculated. It was confirmed that the recovery rate in a case where the unlinked polypeptide was not removed was 0.00086%, the recovery rate in a case where the unlinked polypeptide was removed was 0.047%, and the polypeptide bound to the FGFR protein could be acquired with about 50-fold higher efficiency by removing the unlinked polypeptide. This result indicates that the FGFR protein-binding polypeptide can be more comprehensively acquired by removing the unlinked polypeptide.

As described above, it has been found that according to the production method of a nucleic acid display library of the present disclosure, a nucleic acid display library having good performance can be simply produced.

The disclosure of Japanese Patent Application No. 2023-060413 filed on April 3, 2023 is incorporated herein by reference in its entirety. All documents, patent applications, and technical standards described in the present specification are incorporated herein by reference to the same extent as in a case of being specifically and individually noted that individual documents, patent applications, and technical standards are incorporated herein by reference. [Sequence list] International application 23F00023W1JP24008332_4.xml based on International Patent Cooperation Treaty

## Claims

1. A production method of a nucleic acid display library, comprising:
producing a nucleic acid-polypeptide conjugate by expressing a polypeptide from a nucleic acid and linking the nucleic acid to the polypeptide, in which a modification molecule may or may not be present in the nucleic acid-polypeptide conjugate; and
separating the nucleic acid-polypeptide conjugate from a mixture of a polypeptide not linked to the nucleic acid and the nucleic acid-polypeptide conjugate, independently of a nucleotide sequence of the nucleic acid and the modification molecule.

2. The production method of a nucleic acid display library according to claim 1,
wherein the separation of the nucleic acid-polypeptide conjugate includes
bringing the mixture into contact with a solid-phase carrier capable of binding to the nucleic acid of the nucleic acid-polypeptide conjugate, or
adding a solution capable of rendering nucleic acids insoluble.

3. The production method of a nucleic acid display library according to claim 1,
wherein the separation of the nucleic acid-polypeptide conjugate includes bringing the mixture into contact with a solid-phase carrier capable of binding to the nucleic acid of the nucleic acid-polypeptide conjugate.

4. The production method of a nucleic acid display library according to claim 3, further comprising:
eluting the nucleic acid-polypeptide conjugate bound to the solid-phase carrier with water or an aqueous solution.

5. The production method of a nucleic acid display library according to claim 4,
wherein a total concentration of dissolved substances in the aqueous solution is 0.2 M or less.

6. The production method of a nucleic acid display library according to claim 2,
wherein a surface material of the solid-phase carrier is silica, glass, polysaccharides, a carboxy group-containing polymer, a hydroxy group-containing polymer, or hydroxyapatite.

7. The production method of a nucleic acid display library according to claim 2,
wherein the solid-phase carrier is magnetic beads.

8. The production method of a nucleic acid display library according to claim 2,
wherein the solid-phase carrier is magnetic beads coated with silica or magnetic beads coated with a carboxy group.

9. The production method of a nucleic acid display library according to claim 1,
wherein the nucleic acid of the nucleic acid-polypeptide conjugate includes mRNA.

10. The production method of a nucleic acid display library according to claim 9, further comprising:
producing a conjugate of an mRNA/cDNA double-stranded complex and a polypeptide by reverse transcription of the mRNA.

11. A screening method comprising:
producing a nucleic acid display library by the production method of a nucleic acid display library according to any one of claims 1 to 10; and
selecting a nucleic acid-polypeptide conjugate having a target activity from the nucleic acid display library and identifying a nucleotide sequence of a nucleic acid of the selected nucleic acid-polypeptide conjugate.

12. A production method of a polypeptide, comprising:
acquiring a polypeptide having the target activity based on a nucleotide sequence identified by the screening method according to claim 11.
